## Europäisches Patentamt

## European Patent Office

(19)

## Office européen des brevets

(11) Veröffentlichungsnummer: **0 115 276**
**B1**

(12) # EUROPÄISCHE PATENTSCHRIFT

(45) Veröffentlichungstag der Patentschrift:
**22.04.87**

(51) Int. Cl.⁴: **A 61 M 5/14**

(21) Anmeldenummer: **84100190.2**

(22) Anmeldetag: **10.01.84**

(54) **Anordung zur Durchflussregulierung von Flüssigkeit.**

(30) Priorität: **24.01.83 DE 3302214**

(43) Veröffentlichungstag der Anmeldung:
**08.08.84 Patentblatt 84/32**

(45) Bekanntmachung des Hinweises auf die Patenterteilung:
**22.04.87 Patentblatt 87/17**

(84) Benannte Vertragsstaaten:
**CH DE FR GB IT LI SE**

(56) Entgegenhaltungen:
**GB - A - 2 021 290**

(73) Patentinhaber: **Siemens-Elema AB, Röntgenvägen 2, S-171 95 Solna 1 (SE)**

(84) Benannte Vertragsstaaten: **SE**

(73) Patentinhaber: **Siemens Aktiengesellschaft Berlin und München, Wittelsbacherplatz 2, D-8000 München 2 (DE)**

(84) Benannte Vertragsstaaten: **CH DE FR GB IT LI**

(72) Erfinder: **Cewers, Göran, Moellevongsvaegen 89, S-222 40 Lund (SE)**
Erfinder: **Johansson, Jan, Vongavaegen 40, S-241 00 Esloev (SE)**
Erfinder: **Olsson, Sven-Gunnar, Dipl.-Ing., Postloda 652, S-240 17 Soedra Sandby (SE)**

ACTORUM AG

EP 0 115 276 B1

## Beschreibung

Die Erfindung betrifft eine Anordnung zur Durchflussregulierung von Flüssigkeit gemäss einleitendem Teil des Patentanspruches 1.

Bei bekannten Infusionsapparaten wird die Infusionsflüssigkeit einem Patienten über einen Schlauch kontinuierlich zugeführt. Durch den durch die Schwerkraft der abfliessenden Flüssigkeit erzeugten Unterdruck wird aus einem Vorratsbehälter tropfenweise Flüssigkeit nachgefüllt. Die Tropfen können detektiert und zur Regelung der Durchflussmenge benutzt werden. Wie aus der US-PS 4 294 248 dazu bekannt ist, kann auf diese Weise die einem Patienten zuzuführende Flüssigkeitsmenge und auch der Zeitraum, in dem diese Menge zugeführt werden soll, geregelt werden. Zur Veränderung der Durchflussgeschwindigkeit dient dabei eine motorgetriebene Klemmvorrichtung, beispielsweise ein Exzenter, der den Schlauchinnenquerschnitt mehr oder weniger freigibt.

Aus der GB-AI 2 021 290 ist eine motorgetriebene Klemmvorrichtung bekannt, die aus einem Exzenter und aus einem federbelasteten Hebel als Gegenstück besteht. Der Hebel wird dabei durch eine Feder gegen eine Klinke gezogen und nimmt dadurch eine definierte Lage gegenüber dem Exzenter ein. Bei Störsituationen wird die Klinke entrastet und der Hebel durch die gleiche Feder derart in Richtung auf den Exzenter gezogen, dass der Schlauch für die Infusionsflüssigkeit vollständig abgeklemmt wird.

Insbesondere beim Einsatz am Patienten, der durch Pflegepersonal an den Schlauch angeschlossen werden muss, sollte die richtige Bedienung des Infusionsapparates auch diesem Personal möglich sein. Der Erfindung liegt daher die Aufgabe zugrunde, eine Anordnung der eingangs genannten Art möglichst so einfach zu gestalten, dass eine einwandfreie Bedienung ohne grossen Aufwand stets sichergestellt ist und dass damit auch die hohen, im Gesundheitswesen geforderten Sicherheitsanforderungen erfüllt werden. Eine weitere Aufgabe der Erfindung sieht vor, eine Fehlbedienung der Anordnung zur Durchflussregulierung praktisch unmöglich zu machen.

Ein weiteres Ziel der Erfindung ist es, neben der Durchflussregulierung mit Hilfe der erfindungsgemässen Anordnung ohne grossen zusätzlichen Aufwand gleichzeitig weitere Funktionen eines Infusionsgerätes steuern zu können.

Diese Aufgabe wird erfindungsgemäss durch die Merkmale des Patentanspruches 1 gelöst. Durch die den Schlauch umschliessende Führung und die Verwendung des ersten Hebels als Teil dieser Führung wird die einfache und fehlerfreie Bedienung der Anordnung ermöglicht. In einem Arbeitsgang werden durch den ersten Hebel vorhandene Führungen für den Schlauch freigegeben und gleichzeitig ein Spalt zwischen diesen und beispielsweise einem Exzenter so vergrössert, dass der Schlauch einfach und schnell eingelegt werden kann. Wird der erste Hebel in die Ausgangslage zurückbewegt, so werden die Führungen verschlossen, so dass der Schlauch in einer definierten Lage festgehalten und gleichzeitig der Hebel in die richtige Stellung gebracht wird, um den Schlauchinnenquerschnitt mit Hilfe des Exzenters verändern zu können.

Eine vorteilhafte Weiterbildung der Anordnung ergibt sich dadurch, dass der erste Hebel ausserhalb seiner Achse eine Ausnehmung aufweist, in die ein zweiter, mittels eines Griffstückes betätigbarer Hebel bewegbar eingreift. Ausnehmung und zweiter Hebel sollten dabei derart ausgebildet sein, dass dieser in einem bestimmten Winkelbereich frei in der Ausnehmung bewegbar ist, beim Überschreiten dieses Winkelbereiches in den beiden Richtungen jeweils den ersten Hebel in die jeweils andere Lage überführt. Um sicherzustellen, dass der erste Hebel auch in der richtigen Verschluss-Stellung verbleibt, ist in Weiterbildung der Erfindung vorgesehen, dass dieser erste Hebel derart federbelastet ist, dass eine Kraft in Richtung zum Verschliessen der Führung wirkt.

Weiterhin kann vorgesehen sein, dass durch den ersten Hebel ein elektrischer Schalter betätigbar ist. Darüberhinaus kann der zweite Hebel derart federbelastet sein, dass er innerhalb des Winkelbereiches, in dem er frei bewegbar ist, zwei definierte Lagen einnehmen kann. Der zweite Hebel kann auf diese Art und Weise praktisch in drei definierte Stellungen gebracht werden. In der einen Stellung gibt der erste Hebel die Führungen frei, so dass der Schlauch eingelegt oder herausgenommen werden kann. In der zweiten Stellung sind durch die auf den ersten Hebel einwirkende Federkraft die Führungen verschlossen und der erste elektrische Schalter betätigt. In dieser Stellung ist es praktisch möglich, das gesamte System noch einmal zu kontrollieren, ohne dass bereits der Durchfluss freigegeben ist. In der dritten Stellung betätigt der zweite Hebel einen weiteren elektrischen Schalter. Bei diesem handelt es sich beispielsweise um einen Lageschalter für einen Motor zum Betreiben des Exzenters. Solange dieser Schalter nicht betätigt wird, befindet sich der Exzenter in einer derartigen Lage, dass der Schlauch durch diesen vollständig abgeklemmt wird. Erst beim Betätigen dieses Schalters wird der Exzenter freigegeben und kann nunmehr eine der gewünschten Durchflussmenge entsprechende Stellung einnehmen. Auch der zweite Hebel kann federbelastet sein. Dabei kann die aufgebrachte Federkraft einmal die Verschlussbewegung und einmal die Öffnungsbewegung des ersten Hebels unterstützen.

Eine die Sicherheit der Anordnung und damit des gesamten Infusionsgerätes wesentlich erhöhende Weiterbildung sieht vor, dass im Bereich des Gegenstückes zusätzlich ein drehbar gelagerter Klemmhebel angeordnet ist, zwischen dem und dem Gegenstück der Schlauch hindurchgeführt ist. Über eine Sperre wird dieser Klemmhebel dabei während des üblichen Betriebes in einer Stellung gehalten, dass der Flüssigkeitsdurchsatz durch den Schlauch nicht behindert wird. In Störsituationen, in denen der Flüssigkeitsdurchsatz abrupt unterbunden werden muss, um keine Gefahr für den zu behandelnden Patienten aufkommen zu lassen, wird diese Sperre aufgehoben und der Klemmhebel mit grosser Kraft gegen den Schlauch gedrückt, so dass der Flüssigkeitsdurchsatz schlagartig unterbunden wird. Ein denkbarer Störfall wäre beispielsweise das Versagen des Exzenters oder auch ein totaler Stromausfall.

Um insbesondere bei batteriebetriebenen Geräten den Energieverbrauch möglichst klein zu halten, ist vorgesehen, dass die Sperre aus einem durch eine Feder in Sperrstellung gehaltenen Zapfen besteht, der mittels eines Elektromagneten aus dieser Stellung herausgeführt werden kann. Elektrische Energie muss in diesem Fall also nur zum Entriegeln der Sperre aufgewandt werden. Um auch bei Ausfall der Batterien noch das sichere Funktionieren der Sperre zu gewährleisten, kann ein Kondensator vorgesehen sein, dessen gespeicherte Ladung praktisch ein ausreichendes Notaggregat darstellt. Eine weitere mögliche Störung können beispielsweise Gasblasen im Flüssigkeitsstrom in der Leitung darstellen. Eine besonders raumgünstige Ausgestaltung erhält man, wenn man einen Blasendetektor in die Anordnung integriert, insbesondere im Bereich einer Führung. Die Signale dieses Blasendetektors können dann wiederum zum Entriegeln der Sperre benutzt werden. Im Hinblick darauf, dass bereits eine erste detektierte Gasblase nicht mehr zum Patienten gelangen darf, ist vorgesehen, den Blasendetektor in Strömungsrichtung vor dem Klemmhebel anzuordnen.

Wiederum unter dem Gesichtspunkt der Energieeinsparung empfiehlt es sich, dass der Klemmhebel derart federbelastet ist, dass in der Lage, in der der Schlauch freigegeben ist, nur ein sehr kleiner Kraftarm für die angreifende Kraft wirksam ist. Dadurch wird auch zum Auslösen der Sperre nur eine minimale Energie verbraucht.

Damit die Anordnung möglichst einfach gestaltet werden kann und das Einlegen und Herausnehmen des Schlauches weiter erleichtert wird, ist vorgeschlagen, dass sämtliche Führungen annähernd auf einer Geraden angeordnet sind. Der Schlauch kann auf diese Weise in gestreckter Lage einfach und schnell eingelegt werden. Um trotzdem dem falschen Einlegen vorzubeugen, sollte die gesamte Anordnung ausserhalb der Führungen und ausserhalb der notwendigerweise freigelassenen Räume für den Exzenter oder den Klemmhebel derart mit Material ausgefüllt sein, dass ein Verlegen des Schlauches auf einem anderen Weg vollständig ausgeschlossen ist.

Anhand einer Zeichnung wird im folgenden die erfindungsgemässe Anordnung beispielhaft jedoch nicht einengend erläutert. Dabei zeigen:

Fig. 1–4 schematisiert einen Ausschnitt aus einem Infusionsgerät mit der erfindungsgemässen Anordnung in Vorderansicht in vier unterschiedlichen Zuständen,

Fig. 5–8 die entsprechenden Rückansichten und

Fig. 9 eine Seitenansicht in Richtung des Pfeiles A gemäss der Fig. 4.

In allen Figuren sind gleiche Elemente mit gleichen Bezugszeichen versehen.

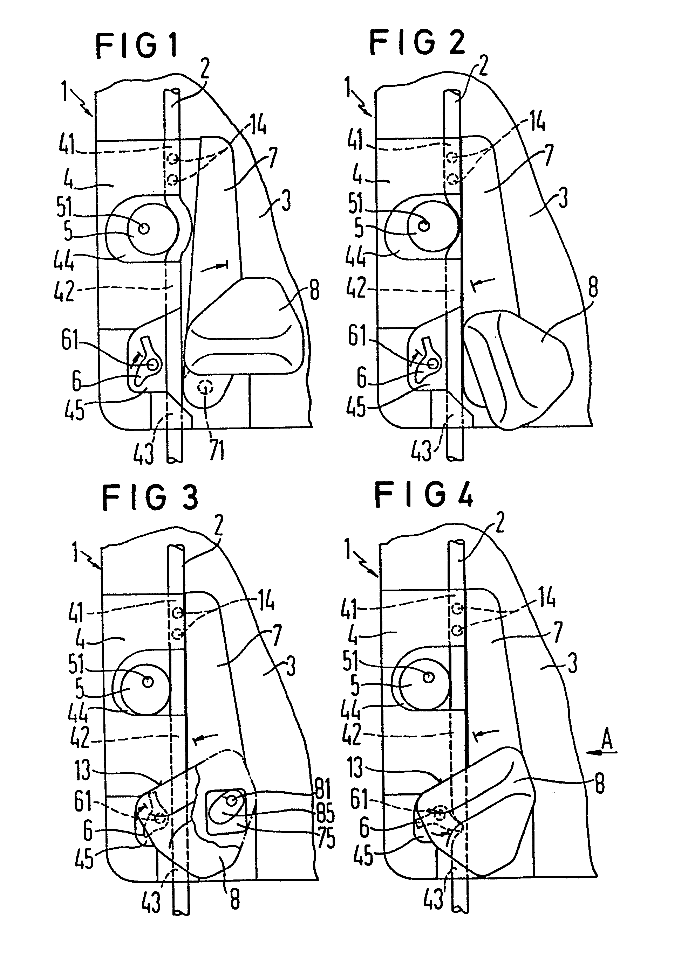

In Fig. 1 ist ein Ausschnitt aus einem Infusionsgerät 1 zur Zufuhr von Infusionsflüssigkeit zu einem nicht dargestellten Patienten über einen Schlauch 2 gezeigt. An der Oberseite kann der Schlauch an ein Vorratsgefäss angeschlossen sein, aus dem Flüssigkeit tropfenweise nachgeführt wird. Die Tropfen können in bekannter Weise mittels eines Detektors registriert und zur Steuerung der Durchflussgeschwindigkeit benutzt werden. Die erfindungsgemässe Anordnung gemäss diesem Ausführungsbeispiel besteht aus einem auf einer Frontplatte 3 des Gerätes 1 angebrachten Kunststoffteil 4, das eine Reihe von Führungsschlitzen 41, 42 und 43 sowie zwei Ausnehmungen 44 bzw. 45 enthält. In der Ausnehmung 44 befindet sich als erster Hebel ein Exzenter 5, dessen Achse mit 51 bezeichnet ist. In der Ausnehmung 45 befindet sich ein Klemmhebel 6. Dem Kunststoffteil 4 gegenüber liegt ein drehbar gelagerter Hebel 7, der als Gegenstück für den Exzenter 5 und den Klemmhebel 6 sowie die Führungen 41, 42 und 43 dient. Die Achse 71 dieses Hebels 7 ist gestrichelt angedeutet. Weiterhin ist in der Fig. 1 ein Griffstück 8 dargestellt, mit dessen Hilfe der Hebel 7 in zwei Lagen einstellbar ist und dass darüberhinaus noch weitere Funktionen erfüllt, die im Zusammenhang mit den anderen Figuren noch näher beschrieben werden.

Wie bereits ausgeführt, kann der Exzenter 5, gesteuert über einen Tropfendetektor und motorgetrieben, in bekannter Weise zur Regulierung des Durchflusses dienen.

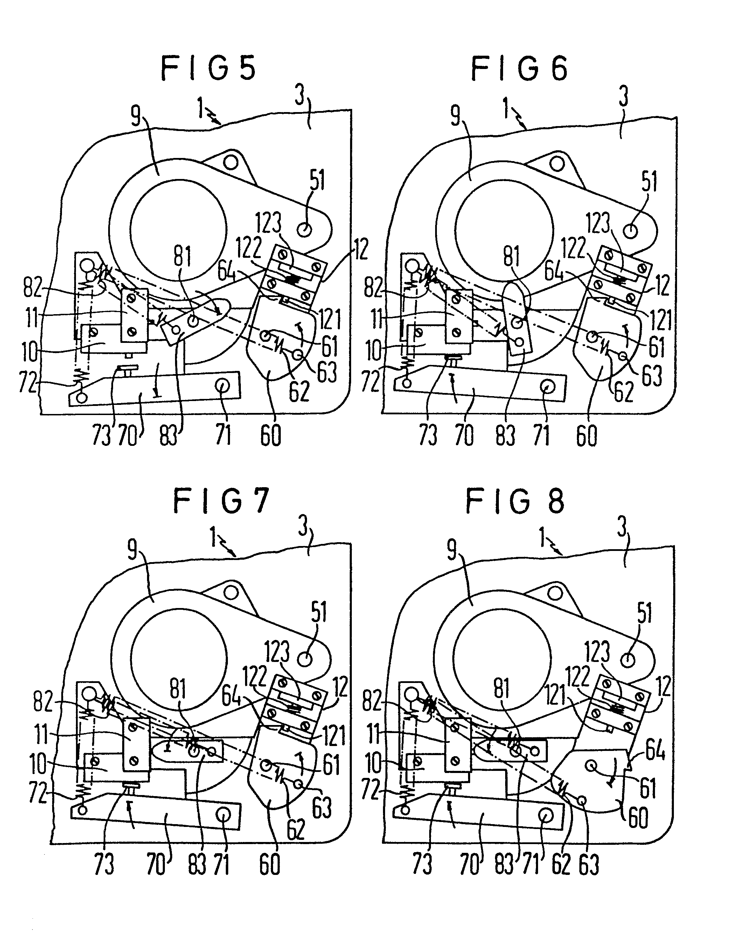

Fig. 5 zeigt in einer Rückansicht diejenigen Teile der Anordnung, die auf der Rückseite der Gehäuseplatte 3 angeordnet sind. In dieser Ansicht ist der Motor 9 zu sehen, der den Exzenter 5 antreibt, von dem in dieser Darstellung nur die durchgehende Achse 51 sichtbar ist. Darüberhinaus ist die durchgehende Achse 61 des Klemmhebels 6 sowie die Achse 81 des Griffstückes 8 sichtbar, letztere ist in der teilweise gebrochenen Darstellung der Fig. 3 in Vorderansicht dargestellt.

Weiterhin sind zwei elektrische Schalter 10 bzw. 11 sowie eine Sperrvorrichtung 12 dargestellt. Wie man dieser Fig. 5 entnehmen kann, setzt sich der Hebel 7 auf der Rückseite der Frontplatte 3 mit einem annährend rechtwinkligen Teil 70 fort. An das Ende dieses Teiles 70 greift eine Feder 72 an, deren Kraft den Hebel 7 in Verschluss-Stellung zieht, d.h. in die Stellung, in der die Führungen 41 bis 43 durch den Hebel 7 abgedeckt sind. Weiterhin dient das Teil 70 dazu, den Schalter 10 über ein Kontaktteil 73 zu betätigen.

Ebenso weist der Klemmhebel 6 auf der Rückseite ein Fortsetzungsteil 60 auf, an das im Punkt 63 eine Feder 62 angreift. Ausserdem weist das Teil 60 am oberen Ende eine Klinke 64 auf, in die ein mit einer Feder 122 belasteter Stift 121 eingreift. Dadurch wird das Teil 60 und damit der Klemmhebel 6 in der in den Fig. 1 bzw. 5 dargestellten Lage gehalten, obwohl die Federkraft der Feder 62 ihn aus dieser Lage herausbewegen möchte. Wie dieser Figur weiter zu entnehmen ist, ist jedoch der Kraftarm für die angreifende Federkraft sehr klein, so dass in dieser Ruhestellung nur eine sehr geringe Kraft aufzuwenden ist, um den Hebel 6 in dieser Lage zu halten. Die Sperre 12 weist darüberhinaus einen Elektromagneten 123 auf, der durch einen kleinen Stromimpuls so aktiviert werden kann, dass der Stift 121 gegen die Kraft der Feder 122 angezogen wird und die Klinke und damit den Klemmhebel 6 freigibt. Die Sperre erfüllt damit eine wesentliche Forderung an die gesamte Anordnung, nämlich extrem energiesparend zu arbeiten, damit auch beim Betrieb mit Batteriespeisung eine möglichst hohe Lebensdauer des Gerätes ermöglicht wird.

Durch einen dem Magneten 123 vorgeschalteten Kondensator kann beispielsweise in einer einfachen Art und Weise eine ausreichende Energie gespeichert werden, um auch bei totalem Stromausfall noch das einwandfreie Auslösen dieser Sperre zu gewährleisten.

Weiterhin ist der Fig. 5 zu entnehmen, dass das Griffstück 8 auf der Rückseite der Gehäuseplatte 3 ein um die Achse 81 drehbares Teil 83 aufweist, auf das ebenfalls mittels einer Feder 82 eine Kraft ausgeübt wird. Mittels dieses Teiles 83 kann – wie in den Fig. 7 und 8 dargestellt – der Schalter 11 betätigt werden. Weiterhin greift die Feder 82 derart an dieses Teil 83 an, dass es in zwei definierte Lagen gebracht werden kann, wobei die Feder die Achse 81 dieses Teiles überstreicht.

Die Fig. 2 bis 4 und 6 bis 8 zeigen die gleiche Anordnung in unterschiedlichen Stellungen. War in Fig. 1 die offene Stellung dargestellt, in der ein Schlauch 2 in die Anordnung eingelegt oder aus dieser herausgenommen werden kann, so zeigen Fig. 2 und 6 eine Stellung, in der die Führungen 41 bis 43 bereits verschlossen sind und der Schalter 10 betätigt wurde, der Schalter 11 jedoch noch nicht.

Die Fig. 3 und 7 zeigen eine Stellung, in der das Griffstück wiederum weitergedreht ist bis an einen Anschlag 13. Durch einen Teil des Griffstückes wird hierbei der Klemmhebel 6 abgedeckt, so dass dieser einem willkürlichen Zugriff entzogen ist. Ausserdem ist in Fig. 3 durch die teilweise gebrochene Darstellung die Achse 81 des Griffstückes 8 sowie ein mit dieser verbundener Hebel bzw. Exzenter 85, der sich in einer Ausnehmung 75 des Hebels 7 befindet, zu sehen. Wie dieser Darstellung zu entnehmen ist, kann sich der Exzenter 85 in der Ausnehmung 75 in einem gewissen Winkelbereich, der etwa durch die Stellungen des Griffstückes 8 in Fig. 3 und 2 festgelegt ist, frei bewegen. Ein Weiterdrehen des Griffstückes 8 über diesen Winkelbereich hinaus verursacht eine Auslenkung des Hebels 7.

In den bisherigen Figuren ist jeweils dargestellt, dass der Klemmhebel 6 gesperrt ist und somit den Flüssigkeitsdurchfluss nicht behindert. In den Fig. 4 und 8 ist nunmehr der Zustand dargestellt, in dem die Sperre kurzzeitig ausgelöst wurde und der Hebel 6 den Schlauch 2 vollständig abklemmt und damit den Flüssigkeitstransport total unterbindet. Wie man der Fig. 8 entnehmen kann, ist in diesem Falle ein relativ grosser Kraftarm für die angreifende Feder 62 wirksam, so dass der Hebel 6 mit einer genügend grossen Kraft gegen den Schlauch gedrückt wird.

Im Bereich der Führung 41 sind zwei Blasendetektoren 14 angeordnet, die beim Auftreten einer Gasblase in der Flüssigkeit die Sperre entriegeln.

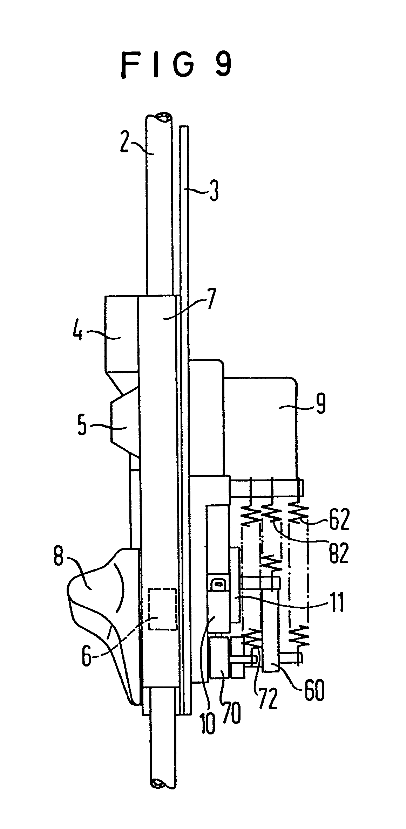

Die letzte Fig. 9 soll in einer schematischen Seitenansicht lediglich verdeutlichen, dass eine raumsparende Anordnung sämtlicher Teile besonders günstig dadurch erreicht werden kann, wenn diese in mehreren Ebenen übereinander angeordnet werden. Sichtbar und zugänglich für den Benutzer sind dabei wiederum nur die Teile auf einer Seite der Gehäusefrontplatte 3. In der Fig. 9 sind das die Teile links von dieser Platte.

Die Funktion der erfindungsgemässen Anordnung ist folgende:

In der in den Fig. 1 und 5 dargestellten Lage kann die Anordnung gewissermassen geladen werden, d.h. der Schlauch 2 kann auf einfache Weise in die Anordnung eingelegt werden. Die Führungen 41 bis 43 sorgen dafür, dass die Lage des Schlauches in der Anordnung eindeutig festgelegt ist. Die Ausnehmungen 44 bzw. 45 sollten vorteilhafterweise so gestaltet sein, dass ein falsches Vorbeiführen des Schlauches an dem Exzenter 5 oder dem Klemmhebel 6 ausgeschlossen wird. Wie Fig. 5 zeigt, sind beide Schalter 10 und 11 noch geöffnet. Wird das Griffstück 8 nunmehr um annähernd 90 Grad geschwenkt, so gibt der Exzenter 85 den Hebel 7 frei, wodurch dieser durch die Feder 72 gegen die Führungen gedrückt wird. Diese werden dadurch verschlossen. Gleichzeitig wird der Schalter 10 betätigt und die Anordnung zwar elektrisch eingeschaltet, jedoch noch nicht in Betrieb genommen, da der Schalter 11 noch nicht betätigt ist. Bei diesem handelt es sich um einen sogenannten Lageschalter, der im nichtbetätigten Zustand dafür sorgt, dass der Motor 9 den Exzenter in eine derartige Lage bringt, dass der Schlauch in diesem Bereich vollständig abgeklemmt wird. Erst wenn das Griffstück 8 weitergedreht und der Schalter 11 betätigt wird, wobei durch das Griffstück gleichzeitig der Klemmhebel 6 abgedeckt wird, wird die Motorsteuerung freigegeben, um den Durchfluss auf einen vorgegebenen Wert einzustellen.

Mit Hilfe dieser erfindungsgemässen Anordnung ist es gelungen, mittels eines einfachen, drehbar gelagerten Griffstückes sicher und eindeutig mehrere Funktionen ausführen zu können, was die Handhabung für Bedienungspersonal erheblich vereinfacht. Weiterhin ist es mit dieser Anordnung gelungen, die Betriebssicherheit entscheidend zu verbessern und somit Risiken für zu behandelnde Patienten durch eine unkontrollierte Zufuhr von Infusionsflüssigkeit oder durch gefährliche Gasblasen zu beseitigen. Ausserdem erfüllt die Anordnung auch die Anforderung, trotz zusätzlicher Sicherheitsvorkehrungen wie beispielsweise den Klemmhebel 6 möglichst energiesparend zu arbeiten.

In dem gezeigten Ausführungsbeispiel bildet der Hebel 7 nicht nur einen Teil der Führungen 41, 42, 43, sondern auch das Gegenstück für den Exzenter 5 und den Klemmhebel 6. Es sind andere Ausführungsformen im Rahmen der Erfindung denkbar, bei denen z.B. ein separates Gegenstück für den Exzenter 5 und/oder der Klemmhebel 6 vorgesehen ist.

**Patentansprüche**

1. Anordnung zur Durchflussregulierung von Flüssigkeit – insbesondere Infusionsflüssigkeit – durch einen Schlauch (2), mit einem in Richtung auf diesen Schlauch bewegbaren Teil, insbesondere mit einem motorgetriebenen Exzenter (5), und einem drehbar gelagerten ersten Hebel (7) als Gegenstück, dadurch gekennzeichnet, dass mindestens eine den Schlauch (2) umschliessende Führung (41) vorgesehen ist und dass der Hebel (7) ein Teil der Führung (41) bildet und mindestens zwei Lagen einnehmen kann, wobei in der einen die Führung (41) freigegeben und in der anderen verschlossen ist.

2. Anordnung nach Anspruch 1, dadurch gekennzeichnet, dass der erste Hebel (7) ausserhalb seiner Achse eine Ausnehmung (75) aufweist, in die ein zweiter, mittels eines Griffstückes (8) betätigbarer Hebel (85) bewegbar eingreift und wobei Ausnehmung (75) und zweiter Hebel (85) derart ausgebildet sind, dass dieser in einem bestimmten Winkelbereich frei in der Ausnehmung bewegbar ist, beim Überschreiten dieses Winkelbereiches in beiden Richtungen jeweils den ersten Hebel (7) in die jeweils andere Lage überführt.

3. Anordnung nach einem der Ansprüche 1 oder 2, dadurch gekennzeichnet, dass der erste Hebel (7) derart federbelastet ist, dass eine Kraft in Richtung zum Verschliessen der Führung (41, 42, 43) wirkt.

4. Anordnung nach einem der Ansprüche 1 bis 3, dadurch gekennzeichnet, dass durch den ersten Hebel (7, 70) ein elektrischer Schalter (10) zum Einschalten der Anordnung betätigbar ist.

5. Anordnung nach einem der Ansprüche 1 bis 4, dadurch gekennzeichnet, dass der zweite Hebel (83, 85) derart federbelastet ist, dass er innerhalb des Winkelbereiches, in dem er frei bewegbar ist, zwei definierte Lagen einnehmen kann.

6. Anordnung nach Anspruch 5, dadurch gekennzeichnet, dass die definierten Lagen durch eine einzige Feder (82) hervorgerufen werden, die so an den zweiten Hebel (83, 85) angreift, dass der Kraftvektor beim Umschalten zwischen den beiden definierten Lagen die Drehachse (81) des zweiten Hebels (83, 85) überstreicht.

7. Anordnung nach Anspruch 5 oder 6, dadurch gekennzeichnet, dass die auf den zweiten Hebel (83, 85) ausgeübte Kraft zumindest teilweise in einer Stellung die Verschlussbewegung und in der anderen Stellung die Öffnungsbewegung des ersten Hebels (7, 70) unterstützt.

8. Anordnung nach einem der Ansprüche 2 bis 7, dadurch gekennzeichnet, dass durch den zweiten Hebel (83, 85) ein Lageschalter (11) betätigbar ist, der im nichtbetätigten Zustand dafür sorgt, dass der Motor (9) den Exzenter (5) in eine derartige Lage bringt, dass der Schlauch (2) vollständig abgeklemmt wird.

9. Anordnung nach einem der Ansprüche 1 bis 8, dadurch gekennzeichnet, dass im Bereich des Gegenstückes 7 ein drehbar gelagerter Klemmhebel (6, 60) angeordnet ist, zwischen dem und dem Gegenstück der Schlauch (2), gegebenenfalls über weitere Führungen (43), hindurchgeführt ist, dass dieser Klemmhebel (6) in einer ersten Lage den Schlauch (2) vollständig abklemmt, dass dieser Hebel (6, 60) federbelastet ist, dass in dieser Lage ein grosser und in einer weiteren Lage, in der der Schlauch (2) freigegeben ist, nur ein sehr kleiner Kraftarm für die angreifende Feder (62) wirksam ist und dass dieser Klemmhebel (6, 60) durch eine Sperre (12) in der weiteren Lage haltbar ist.

10. Anordnung nach Anspruch 9, dadurch gekennzeichnet, dass die Sperre (12) aus einem insbesondere durch eine Feder (122) in Sperrstellung gehaltenen Zapfen (121) besteht, der mittels eines Elektromagneten (123) aus dieser Stellung herausgeführt werden kann.

11. Anordnung nach Anspruch 9 oder 10, dadurch gekennzeichnet, dass das Griffstück (8) derart ausgebildet ist, dass es in der Stellung des zweiten Hebels (83, 85), bei der dieser den entsprechenden elektrischen Schalter (11) betätigt und bei der der erste Hebel (7, 70) alle Führungen (41, 42, 43) verschliesst, den Klemmhebel (6, 60) abdeckt.

12. Anordnung nach einem der Ansprüche 10 oder 11, dadurch gekennzeichnet, dass im Bereich der Führung (41) in Strömungsrichtung der Flüssigkeit gesehen vor dem Klemmhebel (6, 60) ein Blasendetektor (14) angeordnet ist, der in Abhängigkeit von den erhaltenen Signalen den Elektromagneten (123) betätigt.

13. Anordnung nach einem der Ansprüche 1 bis 12, dadurch gekennzeichnet, dass sämtliche Führungen (41, 42, 43) annähernd auf einer Geraden angeordnet sind.

**Claims**

1. An arrangement for the flow control of fluid, in particular of infusion fluid, through a tube (2), comprising a component which can be moved in the direction of the tube, in particular with a motor-driven eccentric (5), and comprising a rotatably mounted first lever (7) as a counterpart, characterised in that there is arranged at least one guide (41) which surrounds the tube (2) and that the lever (7) forms part of the guide (41) and can adopt at least two positions, where in the one the guide (41) is unobstructed and in the other it is closed.

2. An arrangement as claimed in Claim 1, characterised in that outside its axis the first lever (7) has a recess (75), into which a second lever (85), which can be operated by means of a knob (8), movably engages, and the recess (75) and the second lever (85) are designed to be such that said lever is freely movable in the recess in a specific angular region, and respectively transfers the first lever (7) into the respective other position when said angular region is exceeded in both directions.

3. An arrangement as claimed in one of Claims 1 or 2, characterised in that the first lever (7) is spring-loaded in such a manner that a force acts in the direction for closing the guide (41, 42, 43).

4. An arrangement as claimed in one of Claims 1 to 3, characterised in that an electrical switch (10) for connecting the arrangement can be operated by means of the first lever (7, 70).

5. An arrangement as claimed in one of Claims 1 to 4, characterised in that the second lever (83, 85) is spring-loaded in such a manner that it may adopt two distinct positions within the angular region in which it is freely movable.

6. An arrangement as claimed in Claim 5, characterised in that the distinct positions are defined by a single spring (82) which acts upon the second lever (83, 85) in such a manner that the force vector crosses over the axis of rotation (81) of the second lever (83, 85) when switching between the two distinct positions.

7. An arrangement as claimed in Claim 5 or 6, characterised in that the force exerted on the second lever (83, 85) at least partially supports the closing movement of the first lever (7, 70) in the one position and the opening movement in the other position.

8. An arrangement as claimed in one of Claims 2 to 7, characterised in that a position switch (11) is operable by the second lever (83, 85) which in the non-operated state ensures that the motor (9) brings the eccentric (5) into such a position that the tube (2) is completely clamped off.

9. An arrangement as claimed in one of Claims 1 to 8, characterised in that in the region of the counterpart (7) is arranged a rotatably positioned clamping lever (6, 60), the tube (2) being passed between the lever (6, 60) and the counterpart, possibly by means of further guides (43), that said clamping lever (6) completely closes off the tube (2) in a first position, that said lever (6, 60) is spring-loaded, that in this position a large power arm is effective, and in a further position in which the tube (2) is exposed only a very small power arm is effective, for the biassing spring (62), and that said clamping lever (6, 60) can be secured in the further position by means of a barrier (12).

10. An arrangement as claimed in Claim 9, characterised in that the barrier (12) consists of a pin (121) which in particular is held in the locking position by means of a spring (122) and which can be brought out of said position by means of an electro-magnet (123).

11. An arrangement as claimed in Claim 9 or 10, characterised in that the knob (8) is so designed that, in the position of the second lever (83, 85), in which the latter operates the corresponding electric switch (11) and in which the first lever (7, 70) blocks all guides (41, 42, 43), the knob covers the clamping lever (6, 60).

12. An arrangement as claimed in one of Claims 10 or 11, characterised in that in the region of the guide (41), a bubble detector (14) is arranged preceding the clamping lever (6, 60) in the flow direction of the fluid, which detector operates the electro-magnet (123) in accordance with the received signals.

13. An arrangement as claimed in one of Claims 1 to 12, characterised in that all guides (41, 42, 43) are arranged substantially on a straight line.

**Revendications**

1. Dispositif pour régler le débit d'un fluide – notamment d'un liquide de perfusion – dans un tuyau souple (2), ayant une pièce, notamment un excentrique (5) entraîné par un moteur, susceptible d'être déplacée en direction de ce tuyau souple, et un premier levier (7) monté tournant et servant de pièce antagoniste, caractérisé en ce qu'il est prévu au moins un conduit de guidage (41) entourant le tuyau souple (2) et en ce que le levier (7) forme une partie du conduit de guidage (41) et peut prendre au moins deux positions, l'une dans laquelle le conduit de guidage (41) est ouvert, l'autre dans laquelle il est fermé.

2. Dispositif suivant la revendication 1, caractérisé en ce que le premier levier (7) comporte, en dehors de son axe, un évidement (75) dans lequel pénètre, avec possibilité de déplacement, un second levier (85) susceptible d'être manœuvré au moyen d'une pièce de manœuvre (8), l'évidement (75) et le second levier (85) étant tels que celui-ci peut se déplacer librement dans l'évidement dans un plage angulaire déterminée et, au-delà de cette plage angulaire, dans un sens ou dans l'autre, fait passer le premier levier (7) dans l'autre position.

3. Dispositif suivant l'une des revendications 1 ou 2, caractérisé en ce que le premier levier (7) est chargé par un ressort de manière qu'une force s'applique dans la direction de la fermeture du conduit de guidage (41, 42, 43).

4. Dispositif suivant l'une des revendications 1 à 3, caractérisé en ce qu'un interrupteur électrique (10) pour le branchement de l'installation peut être manœuvré par le premier levier (7, 70).

5. Dispositif suivant l'une des revendications 1 à 4, caractérisé en ce que le second levier (83, 85) est chargé par un ressort, de manière à pouvoir prendre deux positions définies dans la plage angulaire dans laquelle il peut se déplacer librement.

6. Dispositif suivant la revendication 5, caractérisé en ce que les positions définies sont établies par un ressort (82) unique qui attaque le second levier (83, 85) de manière que, lors du passage entre les deux positions définies, le vecteur force passe sur l'axe de rotation (81) du second levier (83, 85).

7. Dispositif suivant la revendication 5 ou 6, caractérisé en ce que la force appliquée au second levier (83, 85) facilite au moins en partie le mouvement de fermeture dans une position du premier levier (7, 70) et le mouvement d'ouverture dans l'autre position.

8. Dispositif suivant l'une des revendications 2 à 7, caractérisé en ce que le second levier (83, 85) peut manœuvrer un interrupteur de position (11) qui, à l'état non manœuvré, fait que le moteur (9) met l'excentrique (5) en une position telle que le tuyau souple (2) est pincé complètement.

9. Dispositif suivant l'une des revendications 1 à 8, caractérisé en ce que dans la région de la pièce antagoniste (7) est disposé un levier de pincement (6, 60) monté tournant, le tuyau souple (2) étant guidé, le cas échéant, par d'autres conduits de guidage (43), en ce que ce levier de pincement (6) pince complètement le tuyau souple (2) en une première position, en ce que ce levier (6, 60) est chargé par un ressort, en ce que, dans cette position, le ressort d'attaque (62) applique un grand bras d'attaque de la force et, dans une autre position dans laquelle le tuyau souple (2) est dégagé, qu'un bras d'attaque de la force très petit et en ce que ce levier de pincement (6, 60) peut être maintenu en l'autre position par un dispositif de verrouillage (12).

10. Dispositif suivant la revendication 9, caractérisé en ce que le dispositif de verrouillage (12) est constitué d'un doigt (121) maintenu en la position de verrouillage, notamment par un ressort (122), et pouvant être dégagé de cette position au moyen d'un électroaimant (123).

11. Dispositif suivant la revendication 9 ou 10, caractérisé en ce que la pièce de manœuvre (8) est constituée de façon à recouvrir le levier de pincement (6, 60) en la position du second levier (83, 85) dans laquelle celui-ci manœuvre l'interrupteur (11) électrique correspondant et dans laquelle le premier levier (7, 70) ferme tous les conduits (41, 42, 43).

12. Dispositif suivant l'une des revendications 10 ou 11, caractérisé en ce que dans la région du conduit (41), est disposé en amont, considéré suivant la direction d'écoulement du liquide, du levier de pincement (6, 60), un détecteur de bulles (14) qui actionne l'électroaimant (123) en fonction des signaux obtenus.

13. Dispositif suivant l'une des revendications 1 à 12, caractérisé en ce que l'ensemble des conduits (41, 42, 43) est disposé sensiblement en ligne droite.

FIG 1

FIG 2

FIG 3

FIG 4

FIG 5

FIG 6

FIG 7

FIG 8

# F I G 9